# EUROPÄISCHE PATENTSCHRIFT

(11) **EP 1 251 774 B1**
(45) Veröffentlichungstag und Bekanntmachung des Hinweises auf die Patenterteilung: **05.07.2006**
(21) Anmeldenummer: 00985094.2
(22) Anmeldetag: 25.11.2000
(51) Int. Cl.: A61B 5/022

(54) **BLUTDRUCKMESSGERÄT MIT NEIGUNGSSENSOR**
BLOOD PRESSURE MONITOR WITH INCLINATION SENSOR
TENSIOMETRE AVEC CAPTEUR D'INCLINAISON

(30) Priorität: 29.12.1999 DE 19963633
(43) Veröffentlichungstag der Anmeldung: 30.10.2002
(73) Patentinhaber: Braun GmbH, 61476 Kronberg (DE)
(72) Erfinder: FREUND, Dirk, 65779 Kelkheim (DE); GIERSIEPEN, Martin, 61440 Oberursel (DE); HARTTMANN, Brigitte, 65527 Niedernhausen (DE); HECK, Ulrich, 61440 Oberursel (DE); HOLLINGER, Stefan, 61476 Kronberg (DE); KRESSMANN, Frank, D-65760 Eschborn (DE); RÖNNEBERG, Gerrit, 64289 Darmstadt (DE); SCHNAK, Fred, 61476 Kronberg (DE)
(86) Internationale Anmeldenummer: PCT/EP2000/011769
(87) Internationale Veröffentlichungsnummer: WO 2001/049171

(56) Entgegenhaltungen:
- DE-A- 19 757 974
- DE-U- 29 612 412
- SU-A- 1 825 091
- US-A- 5 042 505
- US-A- 5 574 442
- US-A- 5 778 879

## Beschreibung

Die Erfindung betrifft ein Blutdruckmeßgerät nach dem Oberbegriff von Anspruch 1.

Derartige Blutdruckmeßgeräte haben integriert in einem Gehäuse eine Anzeigeinrichtung zur Anzeige der Blutdruckmeßwerte, eine Steuereinrichtung zur Steuerung der einzelnen Komponenten des Blutdruckmeßgerätes, eine Stromquelle, einer Aufpumpeinrichtung und ein Ventil zum gesteuerten Aufpumpen und Ablassen der Luft in der Blase einer Manschette, einen Drucksensor zur Erfassung eines Drucksignals, eine Auswerteeinrichtung zur Auswertung des Drucksignals sowie eine vorzugsweise innerhalb eines Gehäuses des Blutdruckmeßgerätes angeordnete Neigungserfassungseinrichtung zur Erfassung der Neigung des Blutdruckmeßgerätes relativ zur Horizontalen und zur Abgabe eines der Neigung entsprechenden, elektrischen Neigungssignals und am Gehäuse angeordnet eine Anlegeeinheit zum Anlegen des Drucksensors an ein Körperglied im Handgelenkbereich eines Unterarms,

Blutdruckmessungen am Handgelenk oder an einem Finger leiden häufig an mangelnder Meßgenauigkeit und unzureichender Reproduzierbarkeit. Dies kann zum Teil auf die hohe Empfindlichkeit der Messungen auf Variationen der Meßposition, d.h. auf die individuelle Lage des Handgelenkes bzw. des Fingers relativ zur Lage des Herzens, zurückgeführt werden. Im Falle einer von der Herzhöhe abweichenden Meßposition wird das Meßergebnis durch die hydrostatische Druckdifferenz zwischen Herz und Meßposition um ca. 0,8 mm Hg/cm verfälscht. Eine mangelhafte Lage während eines Meßvorganges führt somit zu einem systematischen Meßfehler.

Es sind schon verschiedene Vorschläge gemacht worden, um im Hinblick auf diese Problematik eine verbesserte Blutdruckmessung zu erreichen. So ist es insbesondere schon vorgeschlagen worden, die Neigung des Unterarmes zur Horizontalen zu erfassen, da diese Neigung bei einer vorgegebenen Position des Ellenbogens, beispielsweise in einer am Oberkörper anliegenden Stellung, ein Maß für die Höhenlage des Handgelenkes und damit für die hydrostatische Komponente des Blutdrucks im Handgelenkbereich ist.

Ein die Neigung des Blutdruckmeßgerätes erfassendes Blutdruckmeßgerät ist z.B. in der DE 296 12 412 U1 beschrieben. Innerhalb des Gehäuses des Blutdruckmeßgerätes ist ein scheibenförmiges Pendel angeordnet, dessen durch ein Fenster des Gehäuses sichtbarer Umfang eine Farbmarkierung aufweist. Diese gibt einen Neigungsbereich an, in dem die Blutdruckmeßwerte als hinreichend korrekt angesehen werden können, weil die Armhaltung einer Handgelenkposition etwa auf Herzhöhe entspricht. Ähnliche, einfache mechanische Lösungen sind auch schon in der japanischen Offenlegungsschrift JP-8-580 (Anmeldenummer 6-145168) oder in der japanischen Veröffentlichung 09038055 A (Anmeldenummer 07196590) beschrieben worden. Das Erfordernis nach direkter Beobachtbarkeit des Pendels durch das neben der Blutdruckwerte-Anzeigevorrichtung angeordnete Fenster führt zu beträchtlichen Baugrößen derartiger Blutdruckmeßgeräte. Auch ist die Handhabung insoweit erschwert, als das Pendel erst ausschwingen muß, um eine zuverlässige Ablesung zu ermöglichen.

Es sind auch schon Blutdruckmeßgeräte mit Neigungsertassungseinrichtungen bekannt geworden, die in der Lage sind, ein elektrisches Neigungssignal zu erzeugen, was unter anderem aus Gründen der leichteren Weiterverarbeitung elektrischer Signale vorteilhaft ist. Ein derartiges Blutdruckmeßgerät ist beispielsweise im US-Patent 5,778,879 gezeigt, wobei jedoch keine Angaben über die Arbeitsweise der Neigungserfassungseinrichtung gemacht werden. In der japanischen Offenlegungsschrift 7-143970 (Anmeldenummer 5-295062) ist ein Blutdruckmeßgerät beschrieben, das als Neigungswinkelsensor einen nicht näher beschriebenen Elektrolytflüssigkeitssensor verwendet, dessen Ausgangsspannung durch Änderung des Widerstandswertes je nach Neigung geändert wird. Derartige Sensoren erfordern eine aufwendige Abdichtung und sollten zur Vermeidung von Zusammensetzungsänderungen der Elektrolytflüssigkeit mit Wechselspannung betrieben werden, was einen erheblichen Ansteuerungsaufwand erfordert.

Aus der US 5,042,505 ist ein elektronisches Sensorgerät bekannt, mit dem der Wechsel einer Winkelposition relativ zu einer ersten Winkelposition des menschlichen Rückgrades vermessen werden kann. Hierzu ist ein optischer Encoder mit einer beweglichen Pendeleinheit vorgesehen, dessen Ausmaß der Rotation bis zu 360° in ein digitales elektrisches Signal in der Form von elektrischen Pulsen je Winkelgrad Rotation des Pendels bestimmbar ist. Diese bekannte Neigungsertassungseinrichtung weist somit ein bewegliches Ausrichtungselement und eine mit dem Ausrichtungselement zusammenwirkende Neigungssensoreinrichtung auf, die mindestens ein mit dem Ausrichtungselement bewegliches Sensorelement und mindestens ein anderes Sensorelement aufweist, wobei die Sensorelemente derart ausgebildet sind, daß das elektrische Neigungssignal aus der Relativstellung der relativ zueinander beweglichen Sensorelemente ableitbar ist.

Aus der SU 1825091 ist ein Neigungssensor zur Vermessung von Eisenbahnschienen bekannt. Die Neigungsmessung stellt ein Relativsignal bereit, das durch die Stellung eines Pendels mit induktiver Meßeinheit gebildet ist.

Aus der US 5,574,442 ist eine Neigungsensor mit einer beweglichen Elektrode, die zwischen zwei starren Elektroden eingreift bekannt. Abhängig vom Neigungswinkel erzeugt die bewegliche Elektrode eine unterschiedliche elektrische Kapazität zwischen den beiden anderen Elektroden.

Ein Blutdruckmeßgerät der eingangs genannten Art ist aus der DE 197 57 974 A1 bekannt.

Der Erfindung liegt die Aufgabe zugrunde, ein gattungsgemäßes Blutdruckmeßgerät zu schaffen, das eine zur Abgabe eines elektrischen Neigungssignals ausgebildete Neigungserfassungseinrichtung hat, die einfach aufgebaut ist und genau und zuverlässig arbeitet.

Zur Lösung dieser Aufgabe schlägt die Erfindung ein Blutdruckmeßgerät mit den Merkmalen von Anspruch 1 vor.

Bei erfindungsgemäßen Blutdruckmeßgeräten hat die Neigungserfassungseinrichtung mindestens ein bewegliches Ausrichtungselement und eine mit dem Ausrichtungselement zusammenwirkende Neigungssensoreinrichtung, die mindestens ein mit dem Ausrichtungselement bewegliches erstes Sensorelement und mindestens ein relativ zu dem ersten Sensorelement bewegliches zweites Sensorelement aufweist, wobei die Sensorelemente derart ausgebildet sind, daß das elektrische Neigungssignal aus der Relativstellung der gegeneinander beweglichen Sensorelemente ableitbar ist. Dabei ist das Ausrichtungselement als beweglicher Körper zu verstehen, der bezogen auf den Schwerkraftvektor, also zur Vertikalen, stets die gleiche Gleichgewichtsstellung anstrebt, wobei die Neigung des Blutdruckmeßgerätes ein Maß für die Neigung des Unterarms ist. Das Ausrichtungselement kann beispielsweise durch eine Flüssigkeitsoberfläche oder durch einen auf einer Flüssigkeit schwimmenden Schwimmkörper gebildet sein, dessen Ausrichtung durch diejenige der Flüssigkeitsoberfläche bestimmt wird. Es ist auch möglich, das Ausrichtungselement als einachsig oder mehrachsig bewegliches Pendel auszubilden, also als ein außerhalb seines Massenschwerpunktes beispielsweise gehäusefest gelagertes Massenelement. Auch auf gekrümmten Flächen abrollende oder nach dem Kreiselprinzip arbeitende Ausrichtungselemente sind möglich. Die Ausnutzung der Relativbeweglichkeit der Sensorelemente und der eindeutigen Zuordnung zwischen Neigungswinkel und Gleichgewichts-Ruhestellung schafft die Möglichkeit, eine Vielzahl von vorzugsweise physikalischen Effekten zur Neigungssignalerzeugung einzusetzen, insbesondere unter Nutzung von elektrischen, elektromagnetischen und/oder magnetischen Feldern und/oder elektromagnetischen und/oder akustischen Wellen, sowie durch Kapazitätsänderungen bei Lageänderung von Kondensatorflächen.

Das mit der Ausrichtungseinheit bewegliche Sensorelement kann beispielsweise mechanisch oder über ein Kraftfeld an die Bewegung des Ausrichtungselementes gekoppelt sein. Vorzugsweise ist dieses Sensorelement fest mit dem Ausrichtungselement verbunden und/oder durch einen Teil, z.B. einen Oberflächenabschnitt des Ausrichtungselementes gebildet. Das andere Sensorelement kann fest mit einem Gehäuse des Blutdruckmeßgerätes oder einer gehäusefest angebrachten Trägerstruktur, wie einer elektronische Bauteile tragenden Platine oder Leiterplatte oder einem Gehäuse der Neigungserfassungseinrichtung, verbunden sein. Die Relativbewegung ergibt sich dann dadurch, daß sich ein Sensorelement mit dem Blutdruckmeßgerät bewegt, während das der Ausrichtungseinheit zugeordnete Sensorelement eine Gleichgewichtsausrichtung bezogen auf den Schwerkraftvektor beibehält bzw. diese anstrebt. Diese Anordnung, bei der die Gleichgewichts-Relativstellung der Sensorelemente vom Neigungswinkel des Blutdruckmeßgerätes bzw. des dieses tragenden Körpergliedes bestimmt wird, kann zur Erzeugung des Neigungssignales genutzt werden.

Bei einer Weiterbildung wird mindestens ein Sensorelement durch einen vorzugsweise gleichmäßig bzw. kreisbogenförmig gekrümmten Bogenabschnitt gebildet. Der Bogenabschnitt kann im wesentlichen zweidimensional bzw. flächenhaft sein oder dreidimensional in Form eines Körpers vorliegen. Das andere Sensorelement ist im Bereich des Bogenabschnitts, entweder in Berührungskontakt mit diesem oder mit Abstand zu diesem in seiner Nähe, angeordnet. Je nach Lage des Blutdruckmeßgerätes steht dann ein eindeutig der Lage zugeordneter Teil oder Teilabschnitt des Bogenabschnittes in Wirkbeziehung zum anderen Sensorelement, so daß das Neigungssignal z.B. über eine vorzugsweise kontinuierliche Eigenschaftsänderung des Bogenabschnitts in Bogenrichtung herbeigeführt werden kann. Beispielsweise kann sich eine Oberflächeneigenschaft des Bogenabschnitts, wie z.B. sein flächenspezifisches oder integrales Reflexionsvermögen für optische Strahlung bzw. Licht, in Bogenrichtung ändern und/oder es kann sich die Form oder mindestens eine Dimension des Bogenabschnitts, beispielsweise seine Breite, in Bogenrichtung ändern. Es ist auch möglich, ein Neigungssignal aus einer neigungswinkelabhängigen Abstandsänderung zwischen den Sensorelementen abzuleiten, indem der z.B. Bogenabschnitt so gestaltet ist, daß er je nach Neigungswinkel mehr oder weniger weit vom anderen Sensorelement entfernt ist.

Bei einer bevorzugten Weiterbildung hat ein Sensorelement, insbesondere eine sensoraktive bzw. in Zusammenarbeit mit dem anderen Sensorelement wirksame Sensorfläche eines Sensorelementes, eine Breite, die in relativer Bewegungsrichtung der Sensorelemente bzw. in Bogenrichtung variiert. Die Variation verläuft vorzugsweise mindestens abschnittsweise linear, so daß ein besonders einfach auswertbares, linear mit dem Neigungswinkel variierendes Neigungssignal erzeugbar ist. Zweckmäßig ist hierfür eine Keilform oder Trapezform des Sensorelementes vorgesehen.

Besonders vorteilhaft ist es, wenn die Variation einer Eigenschaft des Bogenabschnitts bezogen auf die Bogenrichtung oder relative Bewegungsrichtung symmetrisch zu einer Bezugsposition bzw. Nullposition verläuft, die beispielsweise einer horizontalen Ausrichtung des Blutdruckmeßgerätes entspricht. Auf diese Weise kann ohne gesonderten Auswerteaufwand ein Absolutsignal für die Neigung erzeugt werden und das Blutdruckmeßgerät kann gleichermaßen an beiden Körperseiten verwendet werden. Ggf. kann durch weitere Sensorik eine Unterscheidung zwischen Neigungen nach oben und Neigungen nach unten getroffen werden.

Das Konzept von relativ zueinander beweglichen, zusammenwirkenden Sensorelementen kann auf unterschiedliche Weise zur Erzeugung des Neigungssignals genutzt werden. Bei einer Weiterbildung ist das Ausrichtungselement pendelartig ausgebildet und um eine normalerweise gehäusefeste Drehachse drehbar gelagert und eines der Sensorelemente wird durch einen im wesentlichen konzentrisch zur Drehachse liegenden Bogenabschnitt, insbesondere einen Umfangsabschnitt, des Ausrichtungselementes gebildet. Das andere Sensorelement ist, vorzugsweise unbeweglich, im Bereich des Bogenabschnitts angeordnet. Die Nutzung eines Umfangsabschnitts zur Bildung eines Sensorelementes hat unter anderem den Vorteil, daß schon bei kleinen Verstellwinkeln um die Drehachse relativ große Bogenlängen zur Signalbildung genutzt werden, wodurch die Genauigkeit der Messung gesteigert werden kann. Bei einer anderen Weiterbildung ist ein als Sensorelement dienender Bogenabschnitt gehäusefest als Abrollfläche ausgebildet und wirkt mit einem an einem Wälzkörper vorgesehenen anderen Sensorelement zusammen, wobei der Wälzkörper das Ausrichtungselement bildet. Ein Abrollkontakt zwischen den Sensorelementen ermöglicht eine leichte, reibungsarme Gleichgewichtseinstellung des Ausrichtungselementes trotz Berührung der Sensorelemente bzw. der diese tragendenTeile.

Zur Erzeugung des elektrischen Neigungssignales können unterschiedliche Wirkbeziehungen zwischen den Sensorelementen genutzt werden. Bei einer Ausführungsform ist die Neigungssensoreinrichtung als optisch reflexive Neigungssensoreinrichtung ausgebildet, wobei vorzugsweise ein Sensorelement mindestens eine Reflexionsfläche für Optische Strahlung und das andere Sensorelement mindestens eine Strahlungsquelle und mindestens einen Strahlungsdetektor aufweist, wobei die Reflexionsfläche bezogen auf Strahlungsquelle und Strahlungsdetektor so ausgerichtet ist, daß von der Reflexionsfläche reflektierte Optische Strahlung der Strahlungsquelle in den Strahlungsdetektor fällt. Zur neigungswinkelabhängigen Signalerzeugung kann z.B. der flächenspezifische Reflexionsgrad und/oder die Ausdehnung der Reflexionsfläche im Bereich von Strahlungsquelle und/oder Strahlungsdetektor in relativer Bewegungsrichtung variieren. Mit Vorteil können die beispielsweise mindestens eine Leuchtdiode aufweisende Strahlungsquelle und der mindestens einen Phototransistor oder eine Photodiode aufweisende Strahlungsdetektor in einen gemeinsamen Bauteil nach Art einer Reflexionslichtschranke integriert sein. Eine bevorzugte Ausführungsform dieser Art wird später erläutert.

Es ist auch möglich, die Neigungssensoreinrichtung als optisch transmissive Neigungssensoreinrichtung auszubilden. Auch diese kann mindestens eine Strahlungsquelle bzw. einen Strahlungsemitter und mindestens einen Strahlungsdetektor bzw. Strahlungsempfänger aufweisen, die in einem vorzugsweise ähnlichen Spektralbereich arbeiten und einem der Sensorelemente zugeordnet sind. Das andere Sensorelement, insbesondere das schwerkraftabhängig bewegliche, kann ein optisch transmissives Medium aufweisen, das in diesem Spektralbereich eine geeignete Absorptionscharakteristik hat und das beispielsweise durch einen Bogenabschnitt oder Umfangsabschnitt eines Pendels o. dgl. gebildet sein kann. Die Strahlungsquelle kann über eine optische Strecke entweder direkt oder über einen Reflektor in den Strahlungsempfänger strahlen, wobei die geometrische Anordnung so ist, daß die Strahlung auf dem Weg zwischen Strahlungsquelle und Strahlungsdetektor das optisch transmissive Medium mindestens einmal durchstrahlt. Dabei kann die innerhalb des optisch transmissiven Mediums zurückgelegte Strecke vom Neigungswinkel abhängig sein, so daß je nach Neigungswinkel der Strahlungsdetektor mehr oder weniger stark bestrahlt wird. Auch bei einer optisch transmissiven Messung kann die Funktion des Strahlungsemitters und des Strahlungsempfängers in einem einzigen Bauteil vereinigt sein, beispielsweise in einer auf einer Seite eines Pendels o. dgl. anordenbaren Reflexlichtschranke oder einer Gabellichtschranke, bei der Sender und Empfänger auf gegenüberliegenden Seiten des durchstrahlten Mediums angeordnet sein können. Es ist auch möglich, die Strahlung so zu führen, daß das optisch transmissive Medium mehrfach durchstrahlt wird, wodurch ggf. ein besseres Signal/Rauschverhältnis erzielbar ist.

Die genannten optischen Einrichtungen sind vorzugsweise im wesentlichen strahlungsundurchlässig umkapselt, so daß die Messung nicht von Streulicht bzw. Streustrahlung beeinflußt wird. Wenn im Strahlengang zwischen Strahlungsquelle und Strahlungsdetektor zur Umlenkung ein Reflektor vorgesehen ist, so kann dieser durch einen geeigneten Oberflächenabschnitt eines die Anordnung umgebenden, insbesondere optisch strahlungsdichten Gehäuses gebildet sein. Bei nicht strahlungsdichten Gehäusen bzw.

Kapselungen ist eine Vorrichtung zur Restlichtkompensation vorgesehen. Dafür können diesselben Strahlungsquellen und Strahlungsdetektoren eingesetzt werden, wobei die Restlichkompensation durch (elektronisch/optische) Subtraktion von Reflexionen bei zwei verschiedenen Strahlungsintensitäten durchgeführt wird.

Es ist auch möglich, die Transmission und/oder Reflexion von Wellen zum Aufbau einer akustisch arbeitenden Neigungssensoreinrichtung zu nutzen, die beispielsweise mit Ultraschall arbeitet.

Die beschriebenen optisch oder akustisch arbeitenden Neigungssensoreinrichtungen sind unter anderem deshalb bevorzugt, weil das Zusammenwirken der Sensorelemente berührungslos und frei von Rückwirkungskräften erfolgen kann. Damit kann die schwerkraftbedingte Gleichgewichtseinstellung des Ausrichtungselementes kräftemäßig völlig unbeeinflußt von den zur Neigungswinkelmessung genutzten Vorgängen ablaufen. Zudem können Abdichtprobleme, wie sie bei Elektrolytsensoreinrichtungen auftreten können, vermieden werden. Die Einrichtungen haben dadurch eine praktisch unbegrenzte Lebensdauer und können weitgehend verschleißfrei arbeiten.

Es ist auch möglich, die Neigungssensoreinheit als kapazitive Neigungssensoreinheit auszubilden, wobei die Sensorelemente als zusammenwirkende Ladungsträgerflächen wirken können. Das Neigungssignal kann durch eine neigungswinkelabhängige Flächenausdehnungsänderung des durch die Sensorelemente gebildeten Kondensators und/oder durch eine Abstandsänderung der zusammenwirkenden Ladungsträgerflächen herbeigeführt werden. Bei einer Ausführungsform einer kapazitiven Neigungssensoreinrichtung ist das Ausrichtungselement als vorzugsweise einachsig beweglicher Wälzkörper ausgebildet, der zum Abrollen entlang einer vorzugsweise einachsig gekrümmten Wälzfläche der Neigungssensoreinrichtung ausgebildet ist, wobei sich das Neigungssignal in Abhängigkeit von der Position des Wälzkörpers bezogen auf die Wälzfläche einstellt. Der Wälzfläche kann mindestens eine Ladungsträgerfläche der Neigungssensoreinheit zugeordnet sein, wobei vorzugsweise die Breite der Ladungsträgerfläche quer zur Bewegungsrichtung bzw. Wälzrichtung des Wälzkörpers vorzugsweise linear variiert, wozu die Ladungsträgerfläche beispielsweise keilartig oder trapezförmig geformt sein kann. Eine vorteilhafte Ausführungsform dieser Art wird in Zusammenhang mit den Ausführungsbeispielen beschrieben.

Es ist auch möglich, eine induktiv arbeitende Neigungssensoreinrichtung zu schaffen, bei der beispielsweise ein Sensorelement, insbesondere ein Teil des Ausrichtungselementes, als magnetflußleitendes Element ausgebildet ist. Das andere Sensorelement kann mindestens eine elektrische Spule aufweisen, die vorzugsweise einen aus magnetisierbarem Material bestehenden Spulenkern zur Führung und Verstärkung des magnetischen Flusses umgibt. Die Spuleneinrichtung kann zusammen mit dem magnetflußleitenden Element einen im wesentlichen geschlossenen magnetischen Kreis bilden, wobei der magnetische Widerstand des magnetischen Kreises neigungswinkelabhängig sein kann. Die Sensorelemente können berührungslos zusammenwirken, indem zwischen der beispielsweise hufeisenförmig oder kreisbogenförmig gekrümmten Spulenanordnung bzw. deren Spulenkern und dem magnetflußleitenden Element ein Luftspalt verbleibt. Die elektromagnetische Wechselwirkung zwischen den Sensorelementen kann mit Vorteil nach Art einer Wirbelstrombremse dazu genutzt werden, eine Dämpfung der normalerweise pendelnden Bewegung des Ausrichtungselementes bei Bewegung in seine Ruhelage herbeizuführen.

Die erfindungsgemäß mögliche Erzeugung elektrischer Neigungssignale bietet eine Vielzahl vorteilhafter Möglichkeiten zur Weiterverarbeitung des Neigungssignals, die zu einer optimalen Handhabbarkeit des Gerätes und zu einer erhöhten Meßgenauigkeit der Blutdruckbestimmung führen. Das in der Neigungserfassungseinrichtung vorgesehene bewegliche Ausrichtungselement, das z.B. pendelartig oder als Wälzkörper ausgebildet ist, hat ein charakteristisches Schwingungsverhalten, das durch eine Eigenfrequenz charakterisierbar ist. Abhängig von der Art der Bewegung des Patienten wird bei Einnahme der Meßstellung eine Schwingung des Ausrichtungselementes mit mehr oder weniger starker Anfangsamplitude angeregt. Diese Schwingung klingt bis zur Einstellung des Gleichgewichts allmählich ab, was bei ungedämpfter Schwingung längere Zeit benötigen kann. Es ist möglich, die Schwingung des Ausrichtungselementes aktiv beispielsweise mittels Öldämpfung zu dämpfen. Vorteilhaft ermöglicht es die elektrische Neigungssignalerzeugung, ausgehend von einem errechneten oder experimentell bestimmten Schwingungsverhalten des das Ausrichtungselement umfassenden Schwingungssystems aus einem nach einer Neigungsänderung sich periodisch ändernden, gedämpften Neigungssignal einen Schätzwert für die Gleichgewichtslage des Ausrichtungselementes und damit für den Schwerkraftvektor elektronisch oder rechnerisch zu ermitteln, auch wenn die Gleichgewichtslage noch nicht eingenommen ist. Es muß also im Unterschied zu mechanischen Lösungen nicht mit der Ablesung gewartet werden, bis das Ausrichtungselement ausgeschwungen hat. Zur Schätzwertermittlung kann eine adaptive elektronische Filterung des sich während des Ausschwingens periodisch ändernden Neigungssignales durchgeführt werden.

Je nachdem, für welchen Zweck das Neigungssignal verwendet werden soll, ist es auch möglich, auf die Geschwindigkeit oder die Beschleunigung des Ausrichtungselementes zurückzugreifen, was z.B. durch elektronische oder rechnerische Erzeugung der ersten bzw. zweiten zeitlichen Ableitung des Neigungssignales möglich ist. Aufgrund der hierdurch ableitbaren Bewegung bzw. Beschleunigung des Meßgerätes bzw. des Körpergliedes kann festgestellt werden, ob sich ein Anwender generell in einem Zustand ausreichender Ruhe befindet, um eine Messung sinnvoll durchführen zu können. Wird ein erhöhtes Maß an motorischer Aktivität festgestellt, so daß das Blutdruckmeßergebnis möglicherweise nicht repräsentativ ist, so kann die Messung unterbunden und/oder ein Hinweis auf die geringe Aussagekraft der Messung optisch oder akustisch abgegeben werden. Dynamische Fehler, sogenannte Bewegungsartefakte, die beispielsweise durch Zittern oder eine unkontrollierte Armbewegung zustandekommen können, sind dadurch vermeidbar oder zumindest feststellbar.

Das elektrische Neigungssignal ermöglicht auch auf einfache Weise, einen gemessenen Blutdruckwert entsprechend der erfaßten Neigung zu korrigieren und einen entsprechenden, einer Herzhöhenmessung entsprechenden Wert auszugeben. Bevorzugt wird eine im Zusammenhang mit den Ausführungsformen beispielhaft erläuterte Benutzerführung, die zu einer Korrektur der Armhaltung vor der Blutdruckmessung anhalten soll. Dadurch kann die Auswerteelektronik besonders einfach aufgebaut werden und es ist ein zusätzlicher Erziehungseffekt für den Anwender erzielbar. Auch eine im Zusammenhang mit den Ausführungsformen beschriebene vorteilhafte Anordnung und/oder Ausbildung einer Anzeigeeinrichtung, die im wesentlichen nur dann ablesbar ist, wenn das Handgelenk etwa auf Herzhöhe am Oberkörper gehalten wird, dient der Erhöhung der Zuverlässigkeit und Reproduzierbarkeit der Blutdruckmessungen. Durch sie können Bewegungsartefakte verringert und die Herzhöhe bewußt gemacht werden.

Diese und weitere Merkmale gehen außer aus den Ansprüchen auch aus der Beschreibung und den Zeichnungen hervor, wobei die einzelnen Merkmale jeweils für sich allein oder zu mehreren in Form von Unterkombinationen bei einer Ausführungsform der Erfindung und auf anderen Gebieten verwirklicht sein und vorteilhafte Ausführungen darstellen können.

Ein Ausführungsbeispiel der Erfindung ist in den Zeichnungen dargestellt und wird im folgenden näher erläutert. Es zeigen:
- Fig. 1: eine Person mit einer im Handgelenkbereich angebrachten Ausführungsform eines erfindungsgemäßen Blutdruckmeßgerätes sowie eine vergrößerte Ansicht der an er Schmalseite des Handgelenkes angeordneten optischen Anzeige mit Benutzerführung,
- Fig. 2: eine schrägperspektivische Ansicht eines Teils einer mit einem drehbaren Pendel ausgestatteten Neigungserfassungseinrichtung,
- Fig. 3: eine schrägperspektivische Draufsicht auf das in Fig. 2 gezeigte Drehpendel,
- Fig. 4: eine perspektivische Explosionsdarstellung einer anderen Ausführungsform einer optisch reflexiv arbeitenden Neigungserfassungseinrichtung,
- Fig. 5: eine schrägperspektivische Draufsicht auf eine kapazitiv arbeitende Neigungserfassungseinrichtung mit abgenommenem Gehäusedeckel und
- Fig. 6: eine schrägperspektivische Unteransicht der in Fig. 5 gezeigten Neigungserfassungseinrichtung.

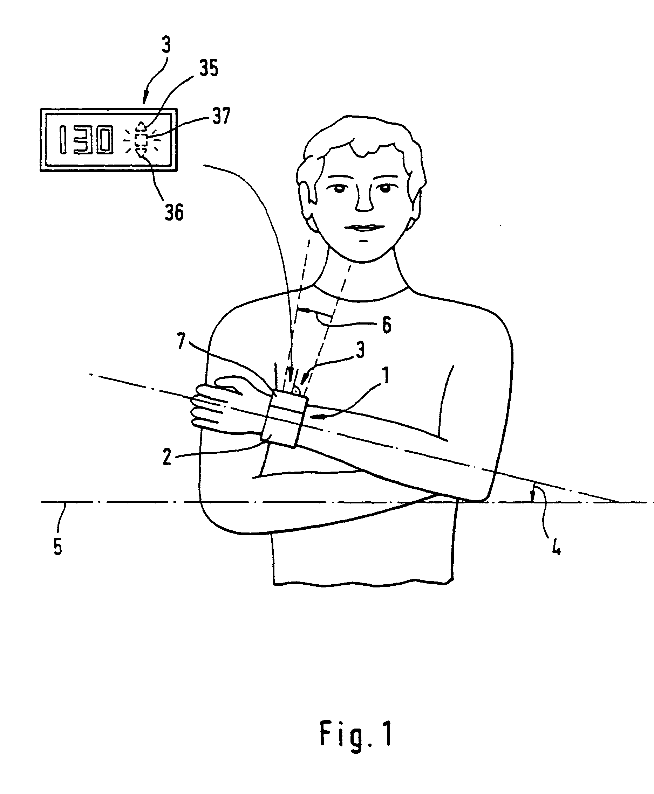

Die in Fig. 1 gezeigte Person trägt im Bereich ihres linken oder rechten Handgelenkes ein Handgelenk-Blutdruckmeßgerät 1, das als Anlegeeinheit eine um das Handgelenk zu legende Manschette aufweist, mit der ein Drucksensor zur Signalaufnahme an die Innenseite des Handgelenkes angelegt werden kann. Es kann sich dabei z.B. um einen kapazitiven oder piezoresistiven Sensor handeln. Die Manschette hat eine integrierte, vorzugsweise mit Luft mittels einer Aufpumpeinrichtung aufpumpbare Blase, mit der die Blutzirkulation durch die am Handgelenkinnenbereich verlaufenden Adern durch Abdrücken unterbrochen werden kann. Während des Luftablassens kann in an sich bekannter Weise z.B. mittels der oszillometrischen Meßmethode der diastolische und der systolische Blutdruck sowie ggf. der mittlere Blutdruck und/oder der Puls bestimmt werden. Im Gehäuse des Blutdruckmeßgerätes ist die Aufpumpeinrichtung, ein Ventil zum Luftablassen, der Drucksensor, eine Anzeigeeinrichtung 3, eine Steuer- bzw- Controllereinheit, eine Stromversorgungsquelle und eine Neigungserfassungseinrichtung integriert. Die Manschette ist mit dem Gehäuse fest verbunden.

Zur optischen Anzeige der Meßwerte ist die Anzeigeeinrichtung 3 vorgesehen, die bei richtig angelegtem Blutdruckmeßgerät im Bereich der daumenseitigen Schmalseite des Handgelenkes liegt, also entlang des Manschettenumfangs um ca. 90° versetzt zur Blase. Bei der in Fig. 1 gezeigten, zur Messung besonders geeigneten Armstellung, bei der der Unterarm in einem ca. 33° betragenden Neigungswinkel 4 zur Horizontalen 5 nach oben angewinkelt ist und die linke Hand den rechten Oberarm umgreift, ist das links oben in Draufsicht gezeigte LCD-Display der Anzeigeeinrichtung 3 dem Kopf der Person direkt zugewandt, so daß der Blick etwa senkrecht auf das Display fällt. Das Display kann so ausgestaltet sein, daß es im wesentlichen nur dann ablesbar ist, wenn die gezeigte richtige Armstellung vorliegt. Dies ist beispielsweise dadurch erreichbar, daß das Display bezogen auf die Anzeigeflächennormale einen sehr kleinen Blickfeldwinkel 6 von beispielsweise 33° +/- 5° oder bis +/- 10° hat, wodurch der Inhalt der Anzeige für den Anwender nicht erkennbar ist, wenn die Blickrichtung außerhalb dieses Raumwinkels liegt. Allein dadurch ist schon eine Benutzerführung geschaffen, die den Anwender dazu anhält, das Blutdruckmeßgerät etwa auf Höhe des Herzens anzuordnen und das Handgelenk an den Oberkörper anzulegen. Das fördert eine besonders ruhige Armhaltung ohne Zittern o. dgl. und eine Verfälschung von Meßergebnissen durch derartige Bewegungsartefakte wird automatisch vermieden.

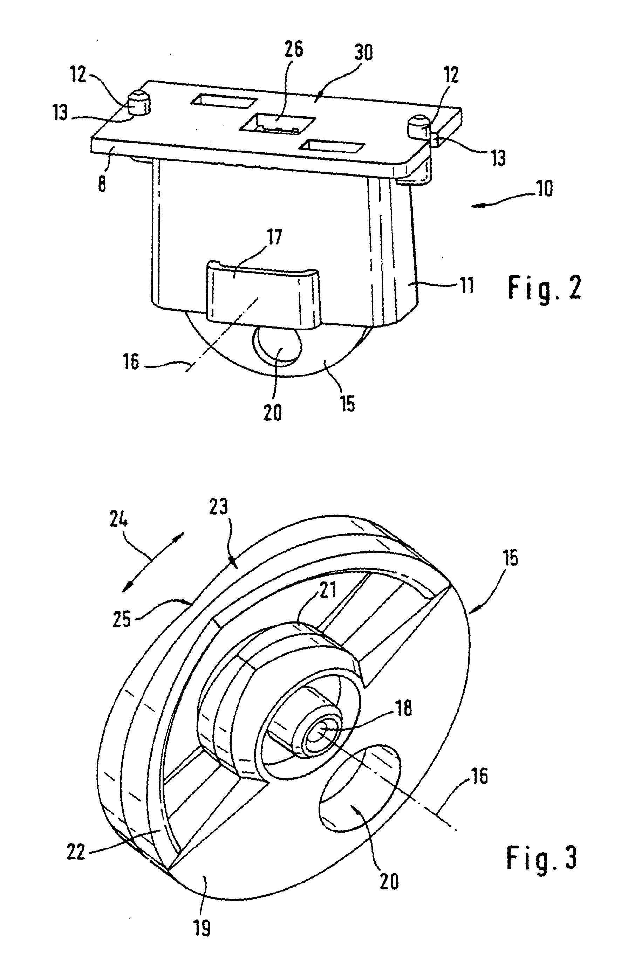

Die optische Anzeige 3 der Anzeigeeinrichtung ist an der Oberseite eines fest mit der Manschette verbundenen Gehäuses 7 des Blutdruckmeßgerätes angeordnet. Direkt unterhalb des Displays ist im Gehäuse, etwa parallel zur Displayebene, eine in Fig. 2 zu erkennende Leiterplatte oder Platine 8 befestigt, die die elektronischen Bauteile der Auswerteeinrichtung und eine im folgenden näher erläuterte Neigungserfassungseinrichtung 10 trägt.

Die Neigungserfassungseinrichtung 10 hat ein flaches, nach oben und unten offenes Kunststoffgehäuse 11, das mittels angeformter Steckstifte 12, die in entsprechende Ausnehmungen 13 der Platine 8 passen, von der dem Display gegenüberliegenden Unterseite der Platine werkzeuglos durch Anstecken an dieser befestigt werden kann. Im Gehäuse 11 ist ein besonders in Fig. 3 gut erkennbares Ausrichtungselement in Form eines einachsigen Drehpendels 15 um eine Drehachse 16 drehbar gelagert. Das Gehäuse 11 hat hierzu im Bereich seitlicher Gehäusewandausbuchtungen 17 auf jeder Seite eine nach unten geöffnete Aufnahme, in die eine durch die zentrische Axialöffnung 18 des Pendels geschobene Welle von unten schnappend eingesetzt werden kann.

Das in Axialansicht kreisförmige Pendel 15 hat einen einstückigen Kunststoff-Pendelkörper mit einem massiven, halbkreisförmigen Pendelunterteil 19 mit einer mittigen, kreisförmigen Aufnahmeöffnung 20, in die eine Metallkugel o. dgl. zur Verlagerung des Masseschwerpunkts des Pendels weit außerhalb der Drehachse 16 schnappend eingesetzt werden kann. Die andere Hälfte des Pendelkörpers wird durch einen konzentrisch zur Drehachse 16 angeordneten inneren Bogenabschnitt 21 und einen in radialem Abstand dazu am Außenumfang des Pendels verlaufenden äußeren Bogenabschnitt 22 gebildet. Dieser bildet einen ca. 180° umfassenden Brückenbogen und hat konzentrisch zur Drehachse 16 eine kreiszylindrisch gekrümmte, radiale Außenfläche oder Umfangsfläche 23. Deren Breite quer zur Umfangsrichtung bzw. parallel zur Drehachse 16 ändert sich in Bogenrichtung 24 kontinuierlich (im Ausführungsbeispiel ändert sich die Breite linear), wobei die Breitenänderung symmetrisch zu einer Nullposition 25 verläuft. Die Nullposition 25, an der zwei aufeinanderzulaufende, sich vom Pendelunterteil zur Nullposition keilförmig auf etwa ein Viertel ihrer axialen Maximalbreite verjüngende Bogenabschnitte zusammenlaufen, liegt diametral zur Achse 16 gegenüber dem Massenschwerpunkt des Pendels, der auf der Verbindungsgeraden zwischen Drehachse 16 und dem Zentrum der Aufnahmeöffnung 20 liegt und bei eingesetzter Metallkugel nahe bei diesem Zentrum angeordnet ist. Wenn das Pendel, wie in Fig. 2 gezeigt, in das Gehäuse 11 eingesetzt ist, so liegt die Außenfläche 23 des Pendels unmittelbar unterhalb der Platine 8 und ist durch ein zentrisches Rechteckfenster 26 der Platine zugänglich bzw. sichtbar, wobei bei horizontaler Platine die Nullposition 25 im Zentrum des Fensters 26 liegt und sich mit der optischen Achse der Reflexionslichtschranke deckt.

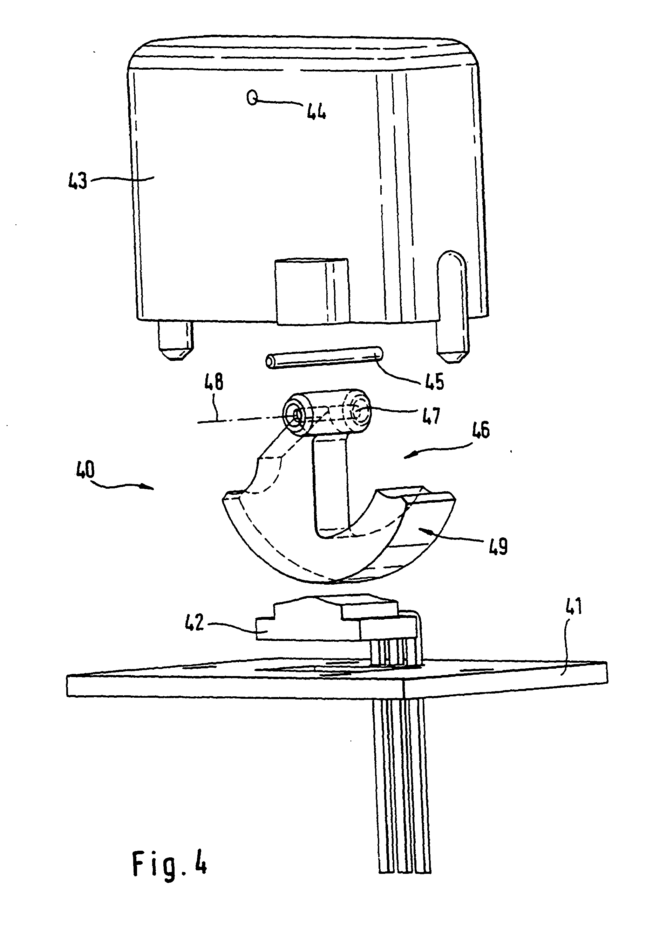

Die Außenfläche 23 des aus hellem, insbesondere weißem Kunststoff gefertigten Pendels 15 dient bei dieser Ausführungsform als ein bewegliches und durch ein Teil des Pendels, nämlich durch die Außenfläche 23 des äußeren Bogenabschnitts gebildetes Sensorelement einer optisch reflexiv arbeitenden Neigungssensoreinrichtung 30. Das damit zusammenwirkende gehäusefeste bzw. mit dem Gehäuse des Blutdruckmeßgerätes bewegliche andere Sensorelement umfaßt eine nicht gezeigte Reflexionslichtschranke (vgl. Ausführungsform nach Fig. 4), die im Bereich des Fensters 26 an der Platine 8 befestigt bzw. befestigbar ist. Der Begriff "Lichtschranke" bezieht sich auf den einer Lichtschranke verwandten Aufbau der Neigungssenoreinrichtung 30 aus Strahlungsquelle und Strahlungsempfänger unabhängig davon, wie die Signale am Strahlungempfänger weiter verwendet werden. Die Lichtschranke hat eine auf die Außenfläche 23 des Pendels 15 gerichtete Strahlungsquelle in Form einer Leuchtdiode und eine direkt daneben am gleichen Bauteil angebrachte, als Strahlungsdetektor dienende Photodiode oder einen Phototransistor, in den das von der Reflexionsfläche 23 diffus reflektierte optische Strahlung der Leuchtdiode einfällt. Dabei sind diese Elemente derart dimensioniert und aufeinander ausgerichtet, daß die Intensität der reflektierten optische Strahlung bzw. die reflektierte Strahlungsmenge in signifikanter Weise von der jeweiligen axialen Breite des im Bereich des Fensters 26 liegenden Teilabschnitts des Bogenabschnitts 23 abhängt. So wird beispielsweise bei horizontaler Ausrichtung der Platine durch den schmalen, nahe der Nullposition liegenden Bereich nur etwa halb so viel Optische Strahlung reflektiert wie bei einer um ca. 45° geneigten Platine, da wegen der keilförmigen Ausbildung des Bogenabschnitts 22 im Bereich der Nullposition 25 die Breite der Außenfläche 23 nur etwa halb so groß ist wie an einer um 45° umfangsversetzten Position. Wegen der zur Nullposition 25 symmetrischen Ausbildung der aufeinanderzulaufenden Trapez- bzw. Keilflächen 23 ergibt sich das gleiche Ausmaß an reflektierter Intensität unabhängig davon, in welche Richtung die Neigung erfolgt, so daß die Neigungserfassung in gleicher Weise funktioniert, wenn das Blutdruckmeßgerät anstatt am linken Handgelenk am rechten Handgelenk angebracht wird.

Die kostengünstig herstellbare Neigungserfassungseinrichtung, die schnell und ohne Zuhilfenahme von Werkzeugen montierbar ist, liefert am Ausgang der Lichtschranke ein leicht auswertbares, stark vom Neigungswinkel idealerweise linear abhängiges elektrisches Neigungssignal. Dank des berührungslosen und kräftefreien Zusammenwirkens der Sensorelemente wird die Gleichgewichtseinstellung des Pendels durch die Sensorik in keiner Weise behindert oder beeinträchtigt. Die Neigungswinkelerfassungseinrichtung kann direkt an der Leiterplatte 8, insbesondere im wesentlichen an ihrer displayabgewandten Unterseite angebracht werden, was einen besonders kompakten Aufbau des Blutdruckmeßgerätes ermöglicht, so daß einerseits eine einfache Befestigung und andererseits eine elektrische Verbindung zwischen der Neigungsensoreinrichtung und der Platine gegeben ist.

Alternativ oder zusätzlich zur äußeren Bogenfläche 23 könnte die ebenfalls symmetrisch keilförmig sich verjüngende Außenfläche des inneren Bogenabschnitts 21 zur Signalerzeugung genutzt werden. Zum Ausgleich von systematischen Nichtlinearitäten der Einheit zur Auswertung der Signale, weist in einer entsprechenden Ausführungsform die äußere Bogenfläche entsprechend angepaßt und demgemäße nichlineare bzw. kontinuierliche Formen bzw. Breitenverläufe auf, d.h. die Geometrie der Neigungssensoreinrichtung kompensiert die systematischen Nichtlinearitäten. Ein im wesentlichen formidentisches Pendel, bei dem beispielsweise der äußere Bogenabschnitt 22 aus einem für sichtbares Licht transparenten, ggf. leicht getrübten Material besteht, kann für ein optisch transmissives Meßsystem genutzt werden, bei dem beispielsweise der dreidimensionale Bogenabschnitt 22 in einer zur Drehachse 16 parallelen Richtung durchstrahlt wird. Entsprechend der Drehstellung des Pendels wird die Länge des durchstrahlten Bereiches und damit die jeweils durchgelassene Intensität unterschiedlich groß sein, was zur Erzeugung eines Neigungssignales genutzt werden kann. Wird beispielsweise die Außenfläche 23 durch eine Metallisierung als Ladungsträgerfläche ausgebildet, so kann im Zusammenwirken mit einer beispielsweise im Fenster 26 mit geringem Abstand zur Umfangsfläche 23 angeordneten weiteren Ladungsträgerfläche ein kapazitiver Sensor geschaffen werden, dessen Kapazität von der Drehstellung des Pendels 15 und damit vom jeweiligen Neigungswinkel in linearer oder einer anderen funktionalen Weise abhängt. Wenn die Außenfläche 23 nicht konzentrisch zur Drehachse verläuft, sondern spiralförmig mit in Umfangsrichtung sich änderndem Radialabstand zur Drehachse, kann eine neigungswinkelabhängige Abstandsänderung zwischen den Sensorelementen erzeugt werden, was z.B. mit einer Kapazitätsmessung auswertbar ist.

Das elektrische Neigungssignal wird bei der gezeigten Ausführungsform und bei den unten beschriebenen Ausführungformen zur Vermeidung lagebedingter Fehlmessungen eingesetzt, indem auf Grundlage des Neigungssignales eine interaktive Benutzerführung erfolgt. Dabei erhält der Anwender auf der Anzeigevorrichtung 3 des Blutdruckmeßgerätes so lange entsprechende optische Anzeichen, bis die den Neigungswinkel bestimmende Unterarmstellung innerhalb eines vorgegebenen Winkeltoleranzbereiches um einen ggf. personenspezifisch vorgebbaren, ggf. individuell einstellbaren Referenzwinkel liegt. Dann ist das Blutdruckmeßgerät 1 in einer Meßposition so nahe bei der Herzhöhe, daß hydrostatisch bedingte Blutdruckmeßfehler ausgeschlossen oder vernachlässigbar sind. Eine Meßwertkorrektur kann entfallen. Die Anzeichen zur Benutzerführung in Richtung zur optimalen Meßlage können am Anfang und/oder während der Druckmessung abgegeben werden. Im Beispiel hat die Anzeigeeinrichtung 3 einen nach oben gerichteten Pfeil 35, einen nach unten gerichteten Pfeil 36 und ein dazwischen liegendes quadratisches Feld 37. Beispielsweise kann der nach oben gerichtete Leuchtpfeil 35 z.B. rot leuchten oder blinken, wenn der Benutzer sein Handgelenk nach oben bewegen soll, um die korrekte Meßposition einzunehmen. Die beispielsweise grüne Anzeige 37 kann bei korrekter Meßposition aufleuchten. Auch eine Benutzerführung beispielsweise durch eine Rotlampe für eine schlechte Meßposition und/oder eine grün aufleuchtende Lampe für eine korrekte Meßposition und/oder ein akustisches Warnsignal bei einer schlechten und/oder "Gut"-Signal bei richtiger Meßposition sind möglich. Es ist auch möglich, die optische Meßwertanzeige erst zu aktivieren, wenn eine korrekte Meßposition vorliegt.

In Fig. 4 ist eine andere Ausführungsform einer optisch reflexiv arbeitenden Neigungssensoreinrichtung 40 gezeigt. Auf einer Leiterplatte oder Platine 41 ist eine Reflexionslichtschranke 42 befestigt, die das gehäusefeste Sensorelement der Einrichtung bildet. Über dem Sensorelement befindet sich eine nach oben geschlossene und nach unten geöffnete Kunststoffkappe 43, die mittels Steckstiften an den Schmalenden auf der Platine 41 befestigbar ist. Im oberen Bereich der Kappe sind an deren Breitseiten miteinander fluchtende Bohrung 44 vorgesehen, die der Befestigung einer durch die Löcher 44 steckbaren Achse oder Welle 45 dienen. Die Achse 45 dient der drehbaren Lagerung eines im zusammengebauten Zustand der Einrichtung innerhalb der Kappe oberhalb der Lichtschranke 42 angeordneten, als Schwinger oder Pendel ausgebildeten Ausrichtungselementes 46, das in einem Nabenabschnitt eine Durchgangsbohrung 47 für die Achse 45 hat. Der einstückig aus hellem Kunststoffmaterial gefertigte Schwinger 46 kann im aufgehängten Zustand um eine durch die Zentralachse der Bohrung 47 gebildete Drehachse 48 pendeln und hat eine konzentrisch zu dieser Drehachse liegende, um diese Achse kreiszylindrisch gekrümmte Umfangsfläche 49, die einen Umfangswinkel von ca. 90° bis 120° einnimmt und dank einer keilförmigen Ausbildung eine in Umfangsrichtung hier linear abnehmende bzw. zunehmende Breite besitzt, die von einem zum anderen Ende etwa auf das Doppelte zunimmt.

In der Ruhelage befindet sich bei horizontaler Platine die trapez- bzw. keilförmige Umfangsfläche 49, die das eine Sensorelement der Sensoreinrichtung bildet, bedingt durch die Lage des Schwerpunktes etwa in der gezeigten Stellung mittig über der als anderes Sensorelement dienenden Reflexionslichtschranke 42. Ein mittelbreiter Abschnitt der Reflexionsfläche 49 liegt der Lichtschranke mit geringem Abstand ohne Berührungskontakt gegenüber. Kippt nun das Blutdruckmeßgerät mit der Sensoreinrichtung nach vorn oder hinten (um eine zur Achse 48 parallele Achse), dann wird die diffus reflektierende Fläche 49 des Schwingers, bedingt durch seine Keilform, im Bereich der die maximale Gesamtbreite der Reflexionsfläche überdeckenden Lichtschranke 42 entsprechend größer oder kleiner. Dies bewirkt, daß die Menge reflektierter optischer Strahlung entsprechend der Breite der Reflexionsfläche bezogen auf die Horizontalstellung entweder mehr oder weniger wird. Durch die Erfassung der reflektierten Strahlungsintensität mittels eines Strahlungsdetektors der Lichtschranke 42 kann damit an deren Ausgang ein elektrisches Neigungssignal erzeugt werden, aus dem sowohl das Ausmaß der Neigung, also der Absolutwinkel, als auch die Richtung der Schrägstellung ableitbar ist.

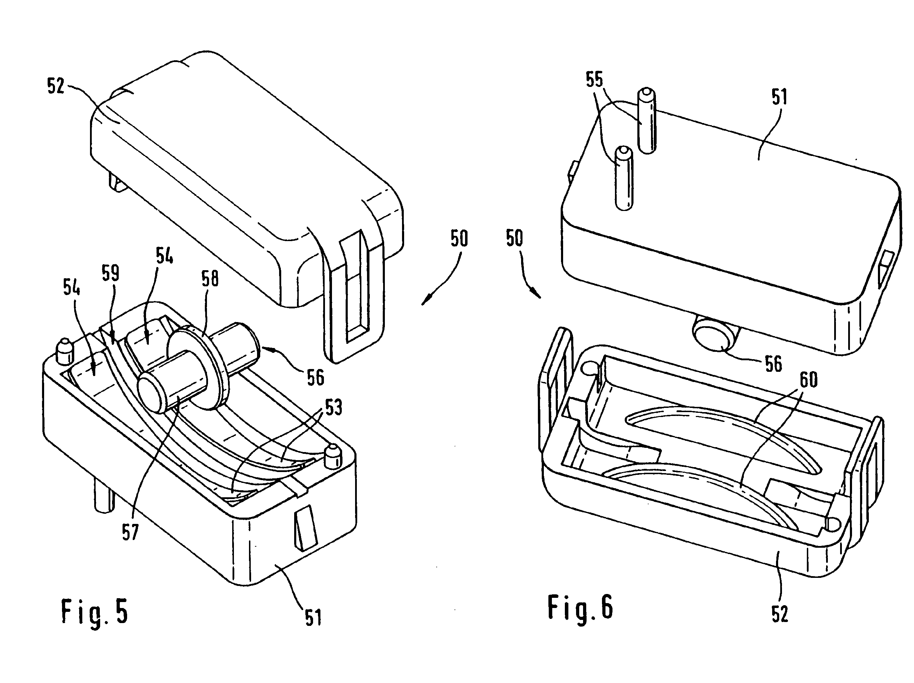

Anhand der Figuren 5 und 6 wird eine Möglichkeit zur elektrisch kapazitiven Erzeugung eines elektrischen Neigungssignales erläutert. Diese Neigungserfassungseinrichtung 50 hat ein Kunststoff-Gehäuseunterteil 51, auf das ein kappenförmiges Kunststoff-Gehäuseoberteil 52 aufschnappbar ist, wozu stirnseitige Laschen des Oberteils 52 mit stirnseitigen Rastnasen des Unterteils zusammenwirken. Zwei schmalseitig am Gehäuseunterteil angeordnete Zapfen greifen dabei in entsprechende Löcher des Oberteils, wodurch die Gehäuseteile gegenseitig zentriert und verdrehgesichert werden. Das Unterteil hat eine kreisbogenförmig gekrümmte, konkavzylindrische Innenseite, auf die parallel und mit Abstand zueinander zwei identische, sich keilförmig verjüngende Metallblechkeile 53 aufgelegt sind. Jeder der Metallkeile bildet an seiner freien Oberseite eine kreiszylindrisch gekrümmte, in Krümmungsrichtung schmäler bzw. breiter werden freie Oberfläche 54. An der gegenüberliegenden Unterseite ist jeweils ein durch den Boden des Gehäuseunterteils hindurchragender Kontaktstift 55 vorgesehen. Eine bei zusammengebauter Vorrichtung innerhalb des Gehäuses angeordnete metallische Rolle 56 hat einen kreiszylindrischen Grundkörper 57, an dem mittig ein scheibenförmiger, umlaufender Steg 58 größeren Durchmessers ausgebildet ist. Die durch die zylindrischen Grundkörperabschnitte beidseits des Steges 58 gebildeten Laufflächen der Rolle sind zur elektrischen Isolation mit einer dünnen Lackschicht überzogen. Der umlaufende Steg der Rolle läuft in einem zwischen den Metallkeilen 53 am Gehäuseunterteil ausgebildeten Führungsschlitz 59 und dient der Seitenführung der Rolle 56 beim Abrollen auf den Metallkeilen 53. In die Kappe 52 sind zwei parallele, kreisbogenförmige Gegenhalter 60 mit einer der Krümmung der Flächen 54 entsprechenden Krümmung integriert, die eine Sicherung gegen Abheben der Rolle 56 von den metallischen Laufflächen 54 bilden.

Die Metallkeile 53 dienen als gehäusefeste Sensorelemente der Sensoreinrichtung, deren freie Oberflächen 54 als Wälzflächen und Ladungsträgerflächen dienen. Der durch die Rolle 56 gebildete Wälzkörper dient als Ausrichtungselement, wobei die der Isolation zugewandten Außenseiten des Grundzylinders 57 ebenfalls Ladungsträgerflächen bzw. Sensorelemente des kapazitiven Sensors bilden. Die Kapazität des Sensors wird im wesentlichen durch die Position des Wälzkörpers 56 auf den Wälzflächen 54 bestimmt und ist im wesentlichen eine Funktion der axialen Länge des praktisch linearen Kontaktbereiches zwischen der Rolle und den Metallkeilen.

In der Ruhelage liegt die Rolle 56 beispielsweise etwa mittig zwischen den Enden der Metallkeile auf den gekrümmten Oberflächen 54 und es stellt sich eine Ausgangskapazität ein. Bei Kippung der Neigungserfassungseinrichtung nach vorne oder hinten rollt der Wälzkörper 56 entsprechend auf den gekrümmten Flächen vor oder zurück. Dabei wird der in Abrollkontakt mit dem Wälzkörper stehende Anteil der keilförmigen Flächen 54 abhängig vom Neigungswinkel größer oder kleiner, so daß die Kapazität des Sensors entsprechend zunimmt oder abnimmt. Durch eine über die Kontaktstifte 55 angeschlossene Auswerteeinrichtung kann die Kapazität der Sensoreinrichtung 50 bestimmt werden. Aus der Kapazität ist ableitbar, in welche Richtung und um wieviel Grad sich der Neigungssensor verkippt bzw. wie weit sich die Rolle bewegt hat.

Es sind zahlreiche Varianten erfindungsgemäßer Blutdruckmeßgeräte möglich. So können als optische Anzeigeeinrichtungen alle Arten von auch bei tragbaren Computereinrichtungen verwendeten Displays eingesetzt werden, z.B. LCD in TN, STN, DSTN, TFT o dgl. anderen Ausführungen.

Gemäß einer weiteren Ausführungsform weist das Blutdruckmeßgerät eine Einstellungseinrichtung auf, mit der die Einstellung des Neigungsbereichs vorgenommen werden kann.

## Patentansprüche

1. Blutdruckmeßgerät mit einer in einem Gehäuse integrierten Anzeigeeinrichtung, einer Steuereinrichtung, einer Stromquelle, einem Ventil, einer Aufpumpeinrichtung, einem Drucksensor zur Erfassung eines Drucksignals, einer Auswerteeinrichtung zur Auswertung des Drucksignals sowie einer Neigungserfassungseinrichtung zur Erfassung der Neigung des Blutdruckmeßgerätes und zur Abgabe eines der Neigung entsprechenden, elektrischen Neigungssignals, die allesamt im Gehäuse aufgenommen sind und einer Anlegeeinheit zum Anlegen des Drucksensors im Handgelenkbereich eines Unterarms, die am Gehäuse angeordet ist, **dadurch gekennzeichnet, daß** die Neigungserfassungseinrichtung mindestens ein bewegliches Ausrichtungselement (15, 46, 56) und eine mit dem Ausrichtungselement zusammenwirkende Neigungssensoreinrichtung hat, die mindestens ein mit dem Ausrichtungselement bewegliches Sensorelement (23, 49, 57) und mindestens ein anderes, Sensorelement (42, 54) aufweist, wobei die Sensorelemente derart ausgebildet sind, daß das elektrische Neigungssignal aus der Relativstellung der relativ zueinander beweglichen Sensorelemente als Absolutsignal ableitbar ist, daß ein Sensorelement (23, 49, 57) fest mit dem Ausrichtungselement verbunden, vorzugsweise durch einen Teil, insbesondere einen Oberflächenabschnitt, des Ausrichtungselementes gebildet ist und daß das andere Sensorelement (42, 54) fest mit einem Gehäuse des Blutdruckmeßgerätes oder einer gehäusefest angebrachten Trägerstruktur verbunden ist, daß mindestens ein Sensorelement durch einen vorzugsweise gleichmäßig gekrümmten Bogenabschnitt (23, 49, 54) gebildet ist und daß das andere Sensorelement (42, 57) derart im Bereich des Bogenabschnitts angeordnet ist, daß jeweils ein eindeutig der Neigung zugeordneter Teil des Bogenabschnittes in Wirkbeziehung zum anderen Sensorelement steht und daß der Bogenabschnitt (23, 49, 54) mindestens eine in Bogenrichtung (24) vorzugsweise linear veränderliche Oberflächeneigenschaft hat, insbesondere ein in Bogenrichtung veränderliches optisches Reflexionsvermögen.

2. Blutdruckmeßgerät nach Anspruch 1, **dadurch gekennzeichnet, daß** der Bogenabschnitt (23, 49, 54) eine in Bogenrichtung (24) veränderliche Breite aufweist.

3. Blutdruckmeßgerät nach einem der vorhergehenden Ansprüche, **dadurch gekennzeichnet, daß** das andere Sensorelement (42, 54) mit einer elektronischen Bauteil tragenden Platine (8) oder einem Gehäuseteil (51) der Neigungserfassungseinrichtung verbunden ist.

4. Blutdruckmeßgerät nach einem der vorhergehenden Ansprüche, **dadurch gekennzeichnet, daß** der Bogenabschnitt in Bogenrichtung derart gestaltet ist, daß sein Abstand zum anderen Sensorelement in Abhängigkeit vom Neigungswinkel vorzugsweise linear variiert.

5. Blutdruckmeßgerät nach einem der vorhergehenden Ansprüche, **dadurch gekennzeichnet, daß** ein Sensorelement (23, 49, 54) mindestens abschnittsweise keilförmig oder trapezförmig gestaltet ist.

6. Blutdruckmeßgerät nach einem der vorhergehenden Ansprüche, **dadurch gekennzeichnet, daß** ein Sensorelement (23, 49) derart mit Abstand in der Nähe des anderen Sensorelementes (42) angeordnet ist, daß die Sensorelemente berührungslos zusammenwirken.

7. Blutdruckmeßgerät nach einem der vorhergehenden Ansprüche, **dadurch gekennzeichnet, daß** die Sensorelemente (23, 49, 42) frei von Rückwirkungskräften zusammenwirken.

8. Blutdruckmeßgerät nach einem der vorhergehenden Ansprüche 1 bis 7, **dadurch gekennzeichnet, daß** ein Sensorelement (54) in Berührungskontakt zu dem anderen Sensorelement (57) steht und / oder beide Sensorelemente zueinander abrollen.

9. Blutdruckmeßgerät nach einem der vorhergehenden Ansprüche, **dadurch gekennzeichnet, daß** bei einem als Bogenabschnitt ausgebildeten Sensorelement die Variation einer Eigenschaft des Bogenabschnitts bezogen auf die Bogenrichtung (24) symmetrisch zu einer Nullposition (25) verläuft, die vorzugsweise einer horizontalen Ausrichtung des Blutdruckmeßgerätes entspricht.

10. Blutdruckmeßgerät nach einem der vorhergehenden Ansprüche, **dadurch gekennzeichnet, daß** das Ausrichtungselement (15, 46) pendelartig ausgebildet und um eine vorzugsweise gehäusefeste Drehachse (16, 48) drehbar gelagert ist, wobei vorzugsweise ein Sensorelement durch einen im wesentlichen konzentrisch zur Drehachse liegenden Bogenabschnitt, insbesondere einen Umfangsabschnitt (23, 49), des Ausrichtungselementes gebildet ist.

11. Blutdruckmeßgerät nach einem der vorhergehenden Ansprüche 1 bis 10, **dadurch gekennzeichnet, daß** ein Sensorelement als, vorzugsweise einachsig, gekrümmte Abrollfläche (54) ausgebildet ist und mit einem anderen Sensorelement (57) zusammenwirkt, das an einem das Ausrichtungselement bildenden Wälzkörper (56) vorgesehen ist.

12. Blutdruckmeßgerät nach einem der vorhergehenden Ansprüche, **dadurch gekennzeichnet, daß** die Neigungssensoreinrichtung unterhalb einer Anzeigeeinrichtung (3) und/oder im wesentlichen unterhalb einer elektronische Bauteile der Auswerteeinrichtung tragenden Platine (8) angeordnet ist

13. Blutdruckmeßgerät nach einem der vorhergehenden Ansprüche, **dadurch gekennzeichnet, daß** eine optisch reflexive Neigungssensoreinrichtung (30, 40) vorgesehen ist, wobei vorzugsweise ein Sensorelement mindestens eine Reflexionsfläche (23, 49) für optische Strahlung und das andere Sensorelement (42) mindestens eine Strahlungsquelle und mindestens einen Strahlungsdetektor aufweist, wobei die Reflexionsfläche bezogen auf Strahlungsquelle und Strahlungsdetektor so ausgerichtet ist, daß von der Reflexionsfläche reflektierte optische Strahlung der Strahlungsquelle in den Strahlungsdetektor fällt.

14. Blutdruckmeßgerät nach einem der vorhergehenden Ansprüche, **dadurch gekennzeichnet, daß** eine optisch transmissive Neigungssensoreinrichtung vorgesehen ist, wobei vorzugsweise ein Sensorelement ein optisch transmissives Medium aufweist und das andere Sensorelement mindestens eine Strahlungsquelle und mindestens einen Strahlungsdetektor aufweist, wobei das optisch transmissive Medium bezogen auf Strahlungsquelle und Strahlungsdetektor so angeordnet ist, daß optische Strahlung der Strahlungsquelle durch das Medium hindurch, entweder direkt oder über mindestens eine Reflexionsfläche, in den Strahlungsdetektor fällt.

15. Blutdruckmeßgerät nach einem der vorhergehenden Ansprüche 13 oder 14, **dadurch gekennzeichnet, daß** die Strahlungsquelle und der Strahlungsdetektor in einen gemeinsamen Bauteil integriert sind, das insbesondere nach Art einer Reflexionslichtschranke (42) oder Gabellichtschranke aufgebaut ist.

16. Blutdruckmeßgerät nach einem der vorhergehenden Ansprüche, **dadurch gekennzeichnet, daß** eine optische Neigungssensoreinrichtung im wesentlichen strahlungsundurchlässig umkapselt ist.

17. Blutdruckmeßgerät nach einem der vorhergehenden Ansprüche, **dadurch gekennzeichnet, daß** die Auswerteeinheit eine Einrichtung zur Ermittlung eines Gleichgewichts-Neigungssignals aufweist, die auf Basis eines errechneten oder gemessenen Schwingungsverhaltens des das Ausrichtungselement umfassenden Schwingungssystems aus einem nach einer Neigungsänderung sich periodisch ändernden, gedämpften Neigungssignal einen Schätzwert für die der neu eingenommenen Neigung entsprechende Gleichgewichtslage des Ausrichtungselementes und für ein zugehöriges Gleichgewichts-Neigungssignal ableitet, bevor diese Gleichgewichtslage erreicht ist.

18. Blutdruckmeßgerät nach einem der vorhergehenden Ansprüche, **dadurch gekennzeichnet, daß** das Blutdruckmeßgerät eine optische Anzeigeeinrichtung (3) aufweist, die bei richtig angelegtem Blutdruckmeßgerät im Bereich einer Schmalseite des Handgelenkes liegt und/oder eine im wesentlichen parallel zur Schmalseite ausgerichtete Anzeigefläche hat.

19. Blutdruckmeßgerät nach einem der vorhergehenden Ansprüche, **dadurch gekennzeichnet, daß** das Blutdruckmeßgerät eine optische Anzeigeeinrichtung (3) aufweist, die derart ausgebildet ist, daß eine Anzeige im wesentlichen nur dann ablesbar ist, wenn eine korrekte Armstellung vorliegt, wobei vorzugsweise die Anzeige bezogen auf eine Anzeigeflächennormale einen Blickfeldwinkel (6) von 33° +/- 5° bis 10° hat.

20. Blutdruckmeßgerät nach einem der vorhergehenden Ansprüche, **dadurch gekennzeichnet, daß** das Blutdruckmeßgerät eine benutzerführende Anzeigeeinrichtung (3) mit Anzeigen aufweist, die derart ansteuerbar sind, daß ein korrekter und/oder ein unkorrekter Neigungsbereich und/oder eine unvorteilhafte Bewegung des Blutdruckmeßgerätes und/oder Anzeigen zur Einnahme einer korrekten Meßhaltung und/oder Hinweise über fehlerhafte Meßbedingungen abgebbar sind, wobei als Anzeigen insbesondere zwei in entgegengesetzte Richtungen gerichtete Pfeile (35, 36) und/oder eine Anzeige (37) zur Anzeige einer korrekten Neigung vorgesehen sind.

## Claims

1. Blood pressure meter having a display device integrated in a housing, a control device, a current source, a valve, a pump device, a pressure sensor for recording a pressure signal, an evaluation device for evaluating the pressure signal and an inclination recording device for recording the inclination of the blood pressure meter and for outputting an electrical inclination signal corresponding to the inclination, all of which are held in the housing, and an application unit for applying the pressure sensor in the wrist region of a forearm, which is arranged on the housing, **characterized in that** the inclination recording device has at least one mobile alignment element (15, 46, 56) and an inclination sensor device which interacts with the alignment element and comprises at least one sensor element (23, 49, 57) mobile with the alignment element, and at least one other sensor element (42, 54), the sensor elements being designed so that the electrical inclination signal can be derived as an absolute signal from the relative position of the sensor elements mobile relative to one another, **in that** one sensor element (23, 49, 57) is firmly connected to the alignment element and is preferably formed by a part, in particular a surface section, of the alignment element, and **in that** the other sensor element (42, 54) is firmly connected to a housing of the blood pressure meter or a support structure fixed to the housing, **in that** at least one sensor element is formed by a preferably uniformly curved arc section (23, 49, 54) and **in that** the other sensor element (42, 57) is arranged in the region of the arc section so that a part, associated uniquely with the inclination, of the arc section is respectively in an active relation with the other sensor element and **in that** the arc section (23, 49, 54) has a surface property varying preferably linearly in the arc direction (24), in particular an optical reflectivity varying in the arc direction.

2. Blood pressure meter according to Claim 1, **characterized in that** the arc section (23, 49, 54) has a width varying in the arc direction (24).

3. Blood pressure meter according to one of the preceding claims, **characterized in that** the other sensor element (42, 54) is connected to a circuit board (8) carrying an electronic component or to a housing part (51) of the inclination recording device.

4. Blood pressure meter according to one of the preceding claims, **characterized in that** the arc section is configured in the arc direction so that its distance from the other sensor element varies preferably linearly as a function of the inclination angle.

5. Blood pressure meter according to one of the preceding claims, **characterized in that** the one sensor element (29, 49, 54) is configured with a wedge shape or trapezoidally at least in sections.

6. Blood pressure meter according to one of the preceding claims, **characterized in that** one sensor element (23, 49) is arranged at a distance in the vicinity of the other sensor element (42) so that the sensor elements interact contactlessly.

7. Blood pressure meter according to one of the preceding claims, **characterized in that** the sensor elements (23, 49, 42) interact freely from reactive forces.

8. Blood pressure meter according to one of Claims 1 to 7, **characterized in that** one sensor element (54) is in touching contact with the other sensor element (57) and/or the two sensor elements roll over each other.

9. Blood pressure meter according to one of the preceding claims, **characterized in that** in the case of a sensor element designed as an arc section, the variation of a property of the arc section relative to the arc direction (24) extends symmetrically with respect to a neutral position (25), which preferably corresponds to a horizontal alignment of the blood pressure meter.

10. Blood pressure meter according to one of the preceding claims, **characterized in that** the alignment element (15, 46) is designed as a pendulum and is mounted rotatably about a rotation axis (16, 48) preferably fixed to the housing, one sensor element preferably being formed by an arc section essentially concentric with the rotation axis, in particular a circumferential section (23, 49), of the alignment element.

11. Blood pressure meter according to one of Claims 1 to 10, **characterized in that** one sensor element is designed as a preferably uniaxially curved rolling surface (54) and interacts with another sensor element (57), which is provided on a roller body (56) that forms the alignment element.

12. Blood pressure meter according to one of the preceding claims, **characterized in that** the inclination sensor device is arranged below a display device (3) and/or essentially below a circuit board (8) carrying electronic components of the evaluation device.

13. Blood pressure meter according to one of the preceding claims, **characterized in that** an optically reflective inclination sensor device (30, 40) is provided, one sensor element preferably comprising at least one reflection surface (23, 49) for optical radiation and the other sensor element (42) comprising at least one radiation source and at least one radiation detector, the reflection surface being aligned relative to the radiation source and radiation detector so that optical radiation from the radiation source reflected by the reflection surface strikes the radiation detector.

14. Blood pressure meter according to one of the preceding claims, **characterized in that** an optically transmissive inclination sensor device is provided, one sensor element preferably comprising an optically transmissive medium and the other sensor element comprising at least one radiation source and at least one radiation detector, the optically transmissive medium being arranged relative to the radiation source and radiation detector so that optical radiation from the radiation source strikes the radiation detector through the medium, either directly or via at least one reflection surface.

15. Blood pressure meter according to one of the preceding Claims 13 and 14, **characterized in that** the radiation source and the radiation detector are integrated in a common component which, in particular, is constructed in the manner of a reflection light barrier (42) or forked light barrier.

16. Blood pressure meter according to one of the preceding claims, **characterized in that** an optical inclination sensor device is encapsulated essentially opaquely to radiation.

17. Blood pressure meter according to one of the preceding claims, **characterized in that** the evaluation unit comprises a device for determining an equilibrium inclination signal, which, from a damped inclination signal varying periodically after an inclination change, on the basis of a computed or measured oscillation behaviour of the oscillation system which comprises the alignment element, derives an estimated value for the equilibrium position of the alignment element, corresponding to the newly assumed inclination, and for an associated equilibrium inclination signal, before this equilibrium position is reached.

18. Blood pressure meter according to one of the preceding claims, **characterized in that** the blood pressure meter comprises an optical display device (3) which, with a correctly applied blood pressure meter, lies in the region of a narrow side of the wrist and/or has a display surface aligned essentially parallel to the narrow side.

19. Blood pressure meter according to one of the preceding claims, **characterized in that** the blood pressure meter comprises an optical display device (3) which is designed so that a display can be read essentially only when there is a correct arm position, the display preferably having a field of view angle (6) of 33° ± 5° to 10° relative to a display surface normal.

20. Blood pressure meter according to one of the preceding claims, **characterized in that** the blood pressure meter comprises a user-guiding display device (3) with displays which can be driven so that a correct and/or incorrect inclination range and/or a disadvantageous movement of the blood pressure meter and/or displays to adopt a correct measurement posture and/or information about faulty measurement conditions can be output, two arrows (35, 36) and/or a display (37) for displaying a correct inclination being in particular provided as displays.

## Revendications

1. Tensiomètre comprenant un dispositif d'affichage intégré dans un boîtier, un dispositif de commande, une source de courant, une valve, un dispositif de gonflage, un capteur de pression destiné à détecter un signal de pression, un dispositif d'interprétation destiné à interpréter le signal de pression ainsi qu'un dispositif de détection de l'inclinaison destiné à détecter l'inclinaison du tensiomètre et à délivrer un signal électrique d'inclinaison correspondant à l'inclinaison, lesquels sont tous logés dans le boîtier, ainsi qu'une unité de port destinée à passer le capteur de pression dans la zone du poignet d'un avant-bras, laquelle est disposé sur le boîtier, **caractérisé en ce que** le dispositif de détection de l'inclinaison possède au moins un élément d'alignement mobile (15, 46, 56) et un dispositif détecteur d'inclinaison qui agit conjointement avec l'élément d'alignement, lequel présente au moins un élément de détection (23, 49, 57) pouvant bouger avec l'élément d'alignement et au moins un autre élément de détection (42, 54), les éléments de détection étant configurés de telle sorte que le signal électrique d'inclinaison peut être dérivé de la position relative des éléments de détection mobiles l'un par rapport à l'autre sous la forme d'un signal absolu, qu'un élément de détection (23, 49, 57) est fixé à demeure avec l'élément d'alignement, de préférence formé par une pièce de l'élément d'alignement, notamment une section de la surface et que l' autre élément de détection (42, 54) est fixé à demeure avec un boîtier du tensiomètre ou avec une structure porteuse montée à demeure sur le boîtier, qu'au moins un élément de détection est formé par une section coudée (23, 49, 54) ayant de préférence une courbure régulière et que l'autre élément de détection (42, 57) est disposé dans la zone de la section coudée de telle sorte qu'une partie explicite de la section coudée associée à l' inclinaison se trouve en relation active avec l' autre élément de détection et que la section coudée (23, 49, 54) possède au moins une propriété de surface variable dans le sens du coude (24), de préférence de manière linéaire, notamment un pouvoir réfléchissant optique variable dans le sens du coude.

2. Tensiomètre selon la revendication 1, **caractérisé en ce que** la section coudée (23, 49, 54) présente un sens du coude (24) de largeur variable.

3. Tensiomètre selon l'une des revendications précédentes, **caractérisé en ce que** l'autre élément de détection (42, 54) est relié avec une platine (8) comportant un composant électronique ou avec une partie de boîtier (51) du dispositif de détection de l'inclinaison.

4. Tensiomètre selon l'une des revendications précédentes, **caractérisé en ce que** la section coudée est configurée dans le sens du coude de telle sorte que son écart par rapport à l'autre élément de détection varie de préférence de manière linéaire en fonction de l'angle d' inclinaison.

5. Tensiomètre selon l'une des revendications précédentes, **caractérisé en ce qu'**un élément de détection (23, 49, 54) présente au moins par sections une forme cunéiforme ou trapézoïdale.

6. Tensiomètre selon l' une des revendications précédentes, **caractérisé en ce qu'**un élément de détection (23, 49) est disposé à un certain écart à proximité de l'autre élément de détection (42) de telle sorte que les éléments de détection interagissent sans contact.

7. Tensiomètre selon l'une des revendications précédentes, **caractérisé en ce que** les éléments de détection (23, 49, 42) interagissent sans forces de contre-réaction.

8. Tensiomètre selon l'une des revendications précédentes 1 à 7, **caractérisé en ce qu'**un élément de détection (54) est en contact physique avec l'autre élément de détection (57) et/ou les deux éléments de détection roulent l'un sur l'autre.

9. Tensiomètre selon l'une des revendications précédentes, **caractérisé en ce que** dans le cas d'un élément de détection réalisé sous la forme d'une section coudée, la variation d'une propriété de la section coudée par rapport au sens du coude (24) est symétrique par rapport à une position zéro (25), laquelle correspond de préférence à un alignement horizontal du tensiomètre.

10. Tensiomètre selon l'une des revendications précédentes, **caractérisé en ce que** l' élément d'alignement (15, 46) est réalisé sous la forme d'un pendule logé de manière à pouvoir pivoter autour d'un axe de rotation (16, 48) de préférence fixé au boîtier, un élément de détection étant de préférence formé par une section coudée disposée de manière essentiellement concentrique par rapport à l'axe de rotation, notamment une section de pourtour (23, 49) de l'élément d'alignement.

11. Tensiomètre selon l'une des revendications précédentes 1 à 10, **caractérisé en ce qu'**un élément de détection est réalisé sous la forme d'une surface de roulement (54) courbée de préférence à un axe et interagit avec un autre élément de détection (57) qui est prévu sur un cylindre (56) formant l'élément d'alignement.

12. Tensiomètre selon l'une des revendications précédentes, **caractérisé en ce que** le dispositif de détection de l'inclinaison est disposé sous un dispositif d'affichage (3) et/ou pour l'essentiel sous une platiné (8) comportant les composants électroniques du dispositif d'interprétation.

13. Tensiomètre selon l'une des revendications précédentes, **caractérisé en ce qu'**il est prévu un dispositif de détection de l'inclinaison (30, 40) optiquement réflectif, un élément de détection présentant de préférence au moins une surface de réflexion (23, 49) pour un rayonnement optique et l'autre élément de détection (42) présentant au moins une source de rayonnement et au moins un détecteur de rayonnement, la surface de réflexion étant alignée par rapport à la source de rayonnement et au détecteur de rayonnement de telle sorte que le rayonnement optique de la source de rayonnement qui est réfléchi par la surface de réflexion pénètre dans le détecteur de rayonnement.

14. Tensiomètre selon l'une des revendications précédentes, **caractérisé en ce qu'**il est prévu un dispositif de détection de l'inclinaison optiquement transmissif, un élément de détection présentant de préférence un support optiquément transmissif et l'autre élément de détection présentant au moins une source de rayonnement et au moins un détecteur de rayonnement, le support optiquement transmissif étant disposé par rapport à la source de rayonnement et au détecteur de rayonnement de telle sorte que le rayonnement optique de la source de rayonnement à travers le support pénètre soit directement, soit par le biais d'au moins une surface de réflexion, dans le détecteur de rayonnement.

15. Tensiomètre selon l'une des revendications précédentes 13 ou 14, **caractérisé en ce que** la source de rayonnement et le détecteur de rayonnement sont intégrés dans un composant commun qui est notamment réalisé sous la forme d'une barrière photoélectrique à réflexion (42) ou d'une barrière photoélectrique à fourche.

16. Tensiomètre selon l'une des revendications précédentes, **caractérisé en ce qu'**un dispositif de détection de l'inclinaison optique est encapsulé essentiellement de manière imperméable au rayonnement.

17. Tensiomètre selon l'une des revendications précédentes, **caractérisé en ce que** l'unité d'interprétation présente un dispositif pour déterminer un signal d'inclinaison d'équilibre qui, sur la base d'un comportement oscillant calculé ou mesuré du système oscillant englobant l'élément d'alignement à partir d'un signal d'inclinaison atténué qui varie périodiquement après un changement d'inclinaison, dérive une valeur estimée pour la position d'équilibre de l'élément d'alignement correspondant à la nouvelle inclinaison adoptée et pour un signal d'inclinaison d'équilibre correspondant avant que cette position d'équilibre soit atteinte.

18. Tensiomètre selon l'une des revendications précédentes, **caractérisé en ce que** le tensiomètre présente un dispositif d'affichage optique (3) qui, lorsque le tensiomètre est bien porté, se trouve dans la zone d'un côté étroit du poignet et/ou possède une surface d'affichage dirigée pour l'essentiel parallèlement au côté étroit.

19. Tensiomètre selon l'une des revendications précédentes, **caractérisé en ce que** le tensiomètre présente un dispositif d'affichage optique (3) qui est configuré de telle sorte qu'un affichage ne peut pour l'essentiel être lu que lorsque le bras se trouve dans une position correcte, l'affichage ayant de préférence une angle de vision (6) de 33° ± 5° à 10° par rapport à une normale à la surface d'affichage.

20. Tensiomètre selon l'une des revendications précédentes, **caractérisé en ce que** le tensiomètre présente un dispositif d'affichage (3) de guidage de l'utilisateur avec des indications qui peuvent être commandées de telle sorte qu'il soit possible de délivrer une plage d'inclinaison correcte et/ou incorrecte et/ou un mouvement désavantageux du tensiomètre et/ou des indications pour adopter une posture de mesure correcte et/ou des notifications relatives à des conditions de mesure incorrectes, les indications prévues étant notamment deux flèches (35, 36) dirigées dans des directions opposées et/ou une indication (37) destinée à indiquer une inclinaison correcte.
